# EUROPÄISCHE PATENTSCHRIFT

(11) **EP 0 798 299 B1**
(45) Veröffentlichungstag und Bekanntmachung des Hinweises auf die Patenterteilung: **27.08.2003**
(21) Anmeldenummer: 97104221.3
(22) Anmeldetag: 13.03.1997
(51) Int. Cl.: C07D 273/04, C07D 251/32, C09D 175/04

(54) **Isocyanattrimerisate und Isocyanattrimerisatmischungen, deren Herstellung und Verwendung**
Isocyanate trimers and mixtures of isocyanate trimers, production and use thereof
Trimères d'isocyanate, mélanges de trimères d'isocyanate, leur préparation et utilisation

(30) Priorität: 26.03.1996 DE 19611849
(43) Veröffentlichungstag der Anmeldung: 01.10.1997
(73) Patentinhaber: BAYER AG, 51368 Leverkusen (DE)
(72) Erfinder: Richter, Frank, Dr., 51373 Leverkussen (DE); Pedain, Josef, Dr., 51061 Köln (DE); Mertes, Harald, Dr., 50670 Köln (DE); Dieris, Carl-Gerd, Dr., 41539 Dormagen (DE)

(56) Entgegenhaltungen:
- EP-A- 0 043 651
- EP-A- 0 166 173
- EP-A- 0 235 388
- EP-A- 0 355 479
- EP-A- 0 379 914
- DE-A- 1 670 666
- C. R. HEBD. SEANCES ACAD. SCI. SER. C, Bd. 277, 1973, Seiten 795-798, XP002034781 A. ETIENNE ET AL.:
- CHEM. BER., Bd. 120, 1987, Seiten 339-344, XP002034782 E. SCHAUMANN ET AL.:

## Beschreibung

Die Erfindung betrifft neue Isocyanattrimerisate und Isocyanattrimerisatmischungen, deren Herstellung und Verwendung

Es ist bekannt, Isocyanate durch Trimerisierung in Isocyanurate (1,3,5-substituierte Hexahydro-s-triazin-2,4,6-trione; Angaben über den Hydrierungsgrad der Heterocyclen wie 'Hexahydro' entfallen im weiteren Text, es werden generell Spezies mit Einfachbindungen im Ring angesprochen) zu überführen. Das 1,3,5-Triphenylderivat, zugänglich beispielsweise durch Trimerisierung von Phenylisocyanat in Gegenwart von Kaliumacetat, wurde bereits 1885 erstmals synthetisiert (A. W. Hofmann, Chem. Ber. 1885, 18, 765 ff.). Obwohl auch auf anderen Wegen möglich (vgl. H. F. Piepenbrink in 'Houben/Weyl, Methoden der Organischen Chemie' 4. Aufl. Bd. VIII, Sauerstoffverbindungen III, G. Thieme Verlag, Stuttgart, 1952, Hrsg. E. Müller, S. 244 ff.), wird die Synthese der Isocyanurate nach wie vor am einfachsten durch Trimerisierung von Isocyanaten durchgeführt.

Insbesondere die durch Trimerisierung technisch in großen Mengen zur Verfügung stehender Diisocyanate wie Toluylendiisocyanat (TDI), Bis(isocyanatophenyl)-methan und Polyphenylen-polymethylen-polyisocyanate, wie sie durch Anilin-Formaldehyd-Kondensation und anschließende Phosgenierung hergestellt werden (MDI), Hexamethylendiisocyanat (HDI), Isophorondiisocyanat (IPDI) und Bis(isocyanatocyclohexyl)methan (H₁₂MDI, Desmodur® W) zugänglichen Isocyanurat-Polyisocyanate haben sich als qualitativ hochwertige Rohstoffe u.a. zur Herstellung von Polyurethan-Kunststoffen und -Beschichtungsmitteln bewährt. Darüber hinaus ist die (anteilige) Trimerisierung insbesondere bei aromatischen Polyisocyanaten als Vernetzungsreaktion zum Aufbau hochmolekularer, ggf. geschäumter, Kunststoffe üblich.

Diese Systeme des Standes der Technik weisen einige Nachteile auf. Werden beispielsweise Diisocyanate zur Herstellung von, insbesondere auf dem Lack- und Beschichtungsmittelsektor bedeutungsvollen, Isocyanurat-Polyisocyanaten trimerisiert, ist die (Schmelz)viskosität der resultierenden Polyisocyanate zuweilen recht hoch. Das trifft insbesondere dann zu, wenn mit hohen Umsetzungsgraden bzw. Harzausbeuten gearbeitet wird woraus zuweilen Probleme bei der Verarbeitung bzw. dem Einsatz dieser Produkte resultieren.

Andererseits ist ein hoher Umsetzungsgrad aber aus mehreren Gründen erwünscht. Zum einen ist dies rein ökonomischen Faktoren geschuldet, die insbesondere wegen des Zeit- und Energieaufwandes bei der aus arbeitshygienischen Gründen im Anschluß an die Trimerisierung meist nötigen Monomerenabtrennung ins Gewicht fallen. Andererseits ist mit zunehmendem Umsetzungsgrad an Ausgangsdiisocyanat durch die sukzessive Bildung mehr als nur einen Isocyanuratring enthaltender Produktanteile eine Erhöhung der NCO-Funktionalität (im folgenden: F) der Trimerisate verbunden. Das wiederum ist sehr erwünscht, da man so zu Produkten mit hoher Vernetzungsdichte und, damit verbunden, hoher physikalischer und chemischer Stabilität gelangt. Diese Spezies sollen im folgenden vereinfachend durch die Anzahl der eingebauten Diisocyanatmoleküle n charakterisiert werden (n 3,5,7,...). Ist n = 3 beträgt F = 3, bei n = 5 ist F = 4 usw.. Mit steigendem n steigt aber naturgemäß die (Schmelz)viskosität der Polyisocyanat-Trimerisate an.

Zur Herstellung niedrigviskoser Trimerisate muß man demzufolge entweder die Reaktion bei sehr niedrigem Umsatz abbrechen, um einen möglichst hohen Anteil an 'n = 3'-Trimerisat in der Mischung zu erhalten, oder man trennt die 'n = 3'-Spezies aus Oligomerenmischungen nachträglich, ggf. auch nur in angereicherter Form, ab (vgl. DE-A 3 810 908; WO-A 93/07 183). Weder das eine noch das andere Verfahren ist jedoch unter wirtschaftlichen Aspekten von Vorteil, da einerseits aus niedrigen Umsetzungsraten enorme Verluste in der Harzausbeute resultieren, was, wie bereits weiter oben ausgeführt, einen großen wirtschaftlichen Aufwand bedeutet und andererseits jegliche Trennoperationen, neben den Mehrkosten des Verfahrens, den Zwangsanfall höherviskoser Fraktionen verursachen. Weiterhin ist es bei der technischen Durchführung der Trimerisierung mitunter schwierig, reproduzierbar einheitliche Produkte zu erhalten, wenn die Weiterreaktion bereits nach sehr kurzer Zeit unterbrochen werden muß (Homogenisierungsprobleme, unvollständige Neben- bzw. Folgereaktionen häufig mitverwendeter Cokatalysatoren usw.).

Aus diesem Grunde sind eine Reihe von Stoffen und Verfahren vorgeschlagen worden, die zur Viskositätserniedrigung von (Lack)polyisocyanat(mischung)en beitragen sollen. Zum einen sind dies sogenannte Reaktivverdünner, d.h Stoffe, die eine niedrige Eigenviskosität, üblicherweise unter 300 mPas bei 23 °C, aufweisen und gegenüber Reaktionspartnern der Polyisocyanate, z. B. Polyhydroxylverbindungen, reaktionsfähige Gruppen besitzen. Hierfür haben sich insbesondere Polyisocyanate auf der Basis aliphatischer Diisocyanate (in erster Linie HDI) mit Uretdionstruktur (sog. "Dimerisate") und Allophanatstruktur bewährt (H. J. Laas et al., J. Prakt. Chem. 1994, 336, 196 - 198.).

Dabei ist es für die Viskosität der Polyisocyanatmischung meist unerheblich, ob sie durch konzertierte Bildung der höherviskosen - z. B. Isocyanurat- und niedrigerviskosen - z. B. Allophanat - Strukturanteile hergestellt wurde, oder ob separat hergestellte Produkte, die z. B. im wesentlichen aus reinen Isocyanurat-Polyisocyanaten und reinen Allophanat-Polyisocyanaten bestehen, im nachhinein gemischt werden.

Sowohl Uretdiongruppen enthaltende, als auch Allophanat-Polyisocyanate, sofern sie auf der Basis difunktioneller Ausgangsisocyanate durch Umsetzung mit Monoalkoholen erhalten wurden, sind streng NCO-difunktionell. Allophanate auf Basis höherwertiger Alkoholmischungen hingegen weisen keine Viskositätsvorteile gegenüber Biuret- bzw. Isocyanurat-Polyisocyanaten auf (DE-A 2 729 990). Daraus resultiert zwangsläufig, daß die Funktionalität einer Polyisocyanatmischung durch eine wie auch immer geartete Kontaminierung mit o.g. Spezies erniedrigt wird. Hierbei hat es sich gezeigt, daß man für eine signifikante Viskositätserniedrigung bei HDI-Polyisocyanaten bereits derartig hohe Konzentrationen an difunktionellen Reaktivverdünnern benötigt, daß die Funktionalität der resultierenden Mischung bereits deutlich unter 3 liegt (DE-A 1 9603 736)

Hinzu kommt, daß der Uretdion-Vierring thermolabil ist und bei höherer Temperatur eine Dissoziation in die zugrundeliegenden (Poly)isocyanate eintritt Bei niedrigviskosen Uretdion-Reaktivverdünnern des Standes der Technik, die z.B. nach der Lehre der DE-A 1670720 durch phosphinkatalysierte Dimerisierung von HDI zugänglich sind, setzt diese allmähliche Rückspaltung unter teilweiser Freisetzung von monomerem HDI bereits bei der Trockenschrank-Lagerung bei Temperaturen oberhalb 60 °C langsam ein.

In untergeordnetem Maße, insbesondere bei Temperaturen oberhalb 150°C, trifft dies auch auf Allophanate zu, die in thermisch stabileres Urethan und Isocyanat dissoziieren.

Andererseits lassen sich niedrigviskose aliphatische Polyisocyanate mit optimaler Funktionalität auch durch alternative Aufbaureaktionen, z. B. durch Umsetzung silylierter Alkohole mit Isocyanatoalkansäurechloriden herstellen (Ch. Zwiener, L. Schmalstieg, M. Sonntag, K. Nachtkamp und J. Pedain, Farbe und Lack 1991, 1052 - 1057 und darin zit. Literatur.). Nachteilig hierbei ist, daß Isocyanatoalkansäurechloride technisch nicht zur Verfügung stehen und ihre Handhabung problematisch sein kann. Das Verfahren ließe sich nur mit einem hohen technischen Aufwand realisieren, der durch die erwarteten Vorteile der Produkte, im wesentlichen die niedrige Viskosität der Polyisocyanate, nicht aufgewogen wird.

Weiterhin weisen Isocyanurat-Polyisocyanate mitunter eine ungenügende Verträglichkeit mit bestimmten Polyolen, z.B. solchen mit geringer Polarität, auf (DE-A 38 10 908). Daraus können mitunter Einschränkungen bei der Verwendbarkeit derartiger Systeme, beispielsweise auf dem Lack- und Beschichtungsmittelsektor, resultieren. Nach der Lehre der DE-A 38 10 908 werden diese Nachteile umgangen, indem man die Trimerisation bereits bei geringem Umsatz abbricht und so zu Isocyanurat-Polyisocyanaten mit mindestens 60 Gewichtsprozent an 1,3,5-Tris(6-isocyanatohexyl)isocyanurat gelangt. Diese Verfahrensweise war allerdings weder neu, noch ist sie, wie weiter oben bereits ausgeführt, unter ökonomischen Aspekten von Vorteil.

Es war deshalb Aufgabe der vorliegenden Erfindung qualitativ den Isocyanuratbasierenden Produkten zumindest gleichwertige Systeme zur Verfügung zu stellen, die nicht, bzw. weniger als letztere, mit den vorstehend genannten Nachteilen der Produkte des Standes der Technik behaftet sind.

Diese Aufgabe konnte durch die Bereitstellung der erfindungsgemäßen, neuen Isocyanat-Trimeren(gemische) gelöst werden.

Gegenstand der Erfindung sind Isocyanat-Trimere(ngemische) auf der Basis aliphatischer, cycloaliphatischer, aromatischer bzw. araliphatischer Mono- sowie Polyisocyanate eines NCO-Gehaltes unter 70 %, die in den organischen Resten ggf. weitere Substituenten wie Carbonyl- oder Carboxylgruppen sowie Heteroatome bzw. Heteroatomgruppierungen (Halogen, O, S, N, P, Si, Sn, B) enthalten können, sowie beliebiger Mischungen dieser Isocyanate, dadurch gekennzeichnet, daß die Trimeren(mischungen) anteilig aus Verbindungen vom Isocyanurat- (**A**) sowie vom Iminooxadiazindion-Strukturtyp (**B**) bestehen, wobei die molare Zusammensetzung der Trimeren(mischungen) im Bereich von 70 % **A** und 30 % **B** bis 100 % B liegen kann, daß das molare Verhältnis von Trimerisat (Summe aus **A** und **B**) zu Uretdion größer als 4:1 ist und daß die Produkte einen Gehalt an Uretoniminen **G** unter 10 mol-% aufweisen.

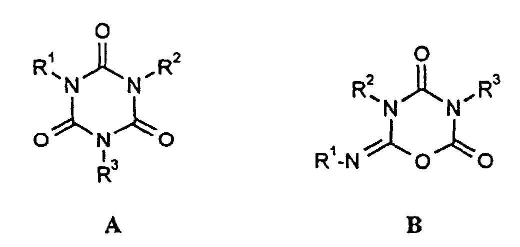

Gegenstand der Erfindung sind weiterhin Mischungen der vorstehend genannten Trimeren(mischungen) mit weiteren Isocyanat-Folgeprodukten, beispielsweise vom Urethan- ('Prepolymer-'), Allophanat-, Harnstoff-, Biuret-, Uretdion- ('Dimerisat-') und/oder Oxadiazintrion-Strukturtyp, wobei diese ihrerseits in den organischen Resten weitere Substituenten, wie NCO-, Carbonyl- oder Carboxylgruppen sowie Heteroatome bzw. Heteroatomgruppierungen (Halogen, O, S, N, P, Si, Sn, B) enthalten können.

Gegenstand der Erfindung ist weiterhin ein Verfahren zur Herstellung der oben genannten Trimeren(mischungen) durch katalytisch induzierte Trimerisierung von Mono- und Polyisocyanat(mischung)en eines NCO-Gehaltes unter 75%, die in den organischen Resten ggf. weitere Substituenten wie Carbonyl- oder Carboxylgruppen sowie Heteroatome bzw. Heteroatomgruppierungen (Halogen, O, S, N, P, Si, Sn, B) enthalten können.

Zur Herstellung der erfindungsgemäßen Isocyanat-Trimeren(gemische) werden Katalysatoren(mischungen) auf der Basis von Hydrogen(poly)fluoriden der Zusammensetzung {M[nF⁻ (HF)ₘ]}eingesetzt, wobei gilt ^{m}/ₙ > 0 und M ein n-fach geladenes Kation bzw. einen n-wertigen Rest darstellt.

Gegenstand der vorliegenden Erfindung ist auch die Verwendung der erfindungsgemäßen Mono- bzw. Polyisocyanat-Trimeren(mischungen) die ggf. NCO-Gruppen, letztere ggf. in blockierter Form, enthalten können, als bzw. zur Herstellung von Wirkstoffe(n), ggf. geschäumte(n) Polyurethan-Kunststoffe(n), Lacken, Beschichtungsmitteln, Klebstoffen sowie Zuschlagstoffe(n).

Der Erfindung liegt die außerordentlich überraschende Beobachtung zugrunde, daß sich Isocyanate ganz allgemein nicht nur auf dem Wege einer ausschließlichen Öffnung der C=N-Doppelbindung, sondern auch über die C=O-Doppelbindung in sechsgliedrige, heterocyclische Systeme überführen lassen.

Diese Beobachtung ist deshalb sehr überraschend, da einer unüberschaubaren Anzahl an Veröffentlichungen über Isocyanat-Folgeprodukte, die durch Öffnung der C=N-Doppelbindung entstehen, bei den Isocyanat-Oligomeren sind das im wesentlichen die Isocyanurate und die Uretdione, nur äußerst wenige Hinweise auf die Existenz der isomeren Iminooxadiazindione **B** gegenüberstehen.

Organisch substituierte Diiminodioxazinone **C** bzw. Triiminotrioxane **D** sind gänzlich unbekannt, mit Ausnahme der Cyanursäure, die zuweilen in der Formelschreibweise von **D**, R¹ -R³= H, dargestellt wird.

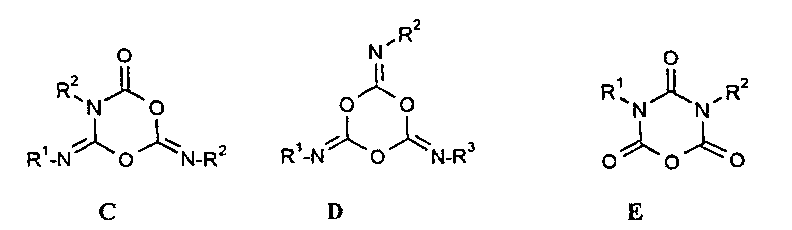

Bisher wurden nur zwei Vertreter der Substanzklasse **B** rein isoliert, das Trimethylderivat (3,5-Dimethyl-2-methylimino-4,6-diketo-1,3,5-oxadiazin), **B,** R¹ - R³ = Me (Chem. Ber. 1927, 60, 295) und das 5-Methyl-2-methylimino-3-phenyl-4,6-diketo-1,3,5-oxadiazin (Chem. Ber 1987, 120, 339).

Weiterhin ist die Bildung von **B** als seltene Nebenreaktion bei der katalysierten Oligomerisierung aliphatischer Diisocyanate bekannt.

So werden bei der Phosphin-katalysierten Uretdionbildung ('Dimerisierung') aliphatischer Diisocyanate "bei höheren Temperaturen und vor allem bei längerer Temperaturbelastung und geringer Katalysatorkonzentration neben Isocyanuraten in wachsender Menge auch andere Nebenprodukte, wie Alkylimino-dialkyloxadiazindione, Carbodiimide und Uretonimine gebildet (DE-A 1670720). Ein molares Verhältnis von Trimerisat (Summe aus **A** und **B**) zu Uretdion von weniger als 5:1 bei der Phosphin-Katalyse kann nicht in Richtung höherer Trimerisatanteile überschritten werden, wenn man den Anfall von Uretoniminen **G** vermeiden will. Der molare Anteil an Iminooxadiazindion **B** im Produkt hingegen, insbesondere aber das Verhältnis von Isocyanurat **A** zu Iminooxadiazindion **B**, bleibt, unabhängig von den Reaktionsbedingungen, nahezu konstant (loc. cit. vgl. Beispiel 1)

Die Uretonimine **G** sind für die Herstellung von Polyurethanen nur bedingt brauchbar, da sie bei noch niedrigerer Temperatur als die strukturell ähnlichen Uretdione in die Edukte dissoziieren ('zurückspalten'); bei **G** also in (z.T. monomeres) Isocyanat und Carbodiimid. Uretonimine liegen bei Zimmertemperatur im dynamischen Gleichgewicht mit Carbodiimiden vor. Liefert ein monomeres (Di)isocyanat die zur Bildung von **G** aus einem beliebigen Carbodiimid nötige NCO-Gruppe, verursacht das entsprechende Uretonimin eine latente Restmonomerenproblematik, was, wie eingangs bereits erwähnt, aus arbeitshygienischen Gründen prohibitiv für einen breiten, gefahrlosen Einsatz entsprechender Produkte auf dem Polyurethan-Kunststoff- und Beschichtungsmittelsektor ist.

Aus diesem Grund wird in der (Patent) Literatur auch immer darauf hingewiesen, die phosphinkatalysierte Oligomerisierung monomerer Diisocyanate bei möglichst niedriger Temperatur durchzuführen (vgl. H. J. Laas et al., J. Prakt. Chem. 1994, 336, 196.).

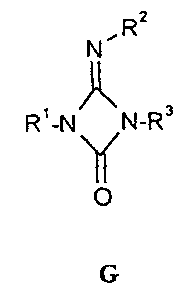

Schließlich wird in der DE-A 3902078 ein Verfahren beschrieben, nach dem durch Trimerisierung (cyclo)aliphatischer Diisocyanate in Gegenwart von Kohlendioxid neben Oxadiazintrionen **E** und Isocyanuraten **A** auch in untergeordnetem Maße, worauf in der genannten Patentschrift auf S. 4, Zeilen 51-52 explizit verwiesen wird, Iminooxadiazindione **B** mitentstehen. Wie den Ausführungsbeispielen der DE-A 39 02078 zu entnehmen ist, liegt der Anteil letzterer, bezogen auf den Trimerisatanteil (Summe aus **A** und **B**, R¹ - R³= (CH₂)₆R⁴ wobei R⁴ durch NCO und/oder Heterocyclen der Struktur **A** und/oder **B** die ihrerseits anstelle von R¹, R² und/oder R³ direkte Verbindungen zur Hexamethylenkette aufweisen, definiert wird) nicht über 25 %. Dasselbe Katalysatorsystem sowie dessen Verwendung zur Herstellung von Isocyanuratgruppen aufweisenden Polyisocyanaten wird in der EP-A 0 355 479 beschrieben.

Bei der HDI-Trimerisierung mit diesem Katalysatorsystem ist auch bei Variation der Herstellungsbedingungen (Cokatalysatoren, Temperatur, Kation etc.) der Anteil von **B** im Trimerisatgemisch (Summe aus **A** und **B**) nie größer als 25 %, in der Regel liegt er unter 20 % (loc. cit. Beispiel 2).

Aus der EP-A 0 355 479 ist bekannt, daß durch die Verwendung der beschriebenen Katalysatorsysteme zur HDI-Trimerisierung bei einer Harzausbeute von 20 bzw. 60 % die dynamische Viskosität, gemessen bei 23 °C (im folgenden η²³), nicht unter 1700 bzw. 35 000 mPas liegt. Die beispielsweise nach der Lehre der DE-A 3806276 durch Katalyse mit quaternären Ammoniumhydroxiden erhältlichen Isocyanurat-Polyisocyanate weisen bei entsprechenden HDI-Trimerisatausbeuten η²³-Werte von ca. 1500 bzw. 10 000 mPas auf (vgl. DE-A 3806276, Beispiele 6-12). Demzufolge stellen die nach der Lehre der EP-A 0 355 479 durch Fluoridkatalyse erhaltenen HDI-Trimerisate bezüglich der Viskosität keine Verbesserung dar.

Es ist deshalb überraschend, daß bei signifikanter Erhöhung des Anteils an **B** in einer Trimerisatmischung bzw. bei alleinigem Vorliegen von **B** eine drastische Reduzierung der Viskosität dieser Produkte erreichbar ist.

Ganz allgemein ist es nahezu unmöglich, Aussagen bezüglich der Viskosität einer bestimmten Verbindung oder Verbindungsklasse aus Analogieschlüssen von anderen Verbindungen bzw. Verbindungstypen herleiten zu wollen. So weist z.B. das 1,3,5-Tris(6-isocyanatohexyl)isocyanat **A**, R¹-R³ = (CH₂)₆-NCO, mit ca. 700 mPas bei 23°C eine erhebliche geringere dynamische Viskosität als das strukturell verwandte, aber nur NCO-difunktionelle 3,5-Bis-(6-isocyanatohexyl)-1-oxadiazintrion **E**, R¹ und R² = (CH₂)₆-NCO, auf, dessen η²³-Wert ca. 1200 mPas beträgt (loc. cit Beispiel 3).

Ein zur Herstellung der erfindungsgemäßen Trimerisate bzw. Trimerisatmischungen geeignetes Katalysatorsystem stellen beispielsweise Hydrogen(poly)-fluoride der allgemeinen Zusammensetzung {M[nF⁻·(HF)ₘ]}, wobei gilt: ^{m}/ₙ > 0 und M ein(e) n-fach geladene(s) Kation(enmischung) bzw. einen oder mehrere, in der Summe n-wertige(r) Rest(e) darstellt, dar. Diese Verbindungen sind teilweise kommerziell erhältlich oder lassen sich in einfacher Weise und in jeder beliebigen Stöchiometrie durch Abmischen entsprechender Fluoride mit der gewünschten HF-Menge herstellen.

Bedenkt man, daß in zahllosen Literaturbeispielen Säuren und Säurederivate zur sicheren 'Abstoppung' von Trimerisierungsreaktionen vorgeschlagen werden (J. Prakt. Chem. **1994**, 336, 185 ff., dort weitere Zitate), ist es außerordentlich überraschend, daß durch die Zugabe der Mineralsäure HF, beispielsweise zu quaternären Ammoniumfluoriden, die katalytische Aktivität der Katalysatoren nicht zerstört, sondern im Gegenteil deren Selektivität entscheidend erhöht wird.

Fluorwasserstoff kann beispielsweise als Lösung in protischen oder aprotischen organischen Lösungsmitteln zugegeben werden. Kommerziell verfügbar sind auch HF-Aminkomplexe, z.B. mit Pyridin oder Melamin. Im Gegensatz zum freien Fluorwasserstoff, der sich physiologisch unangenehm verhält, sind Hydrogenfluoride unproblematisch. Auch ist durch die bekannte Addition von HF an Isocyanate unter Bildung von Carbamoylfluoriden (J. Chem. Soc., **1945**, 864-865) die Anwesenheit von freiem Fluorwasserstoff in den Verfahrensprodukten ausgeschlossen.

Der 'HF-Anteil' in den beschriebenen Katalysatorsystemen kann in weiten Grenzen variieren. D.h., es ist unwesentlich, ob es sich um definierte Monohydrogendifluoride, Dihydrogentrifluoride etc. handelt, die beispielsweise in Form ihrer Kaliumsalze mit der entsprechenden Stöchiometrie bekannt sind (Hollemann-Wiberg, Lehrbuch der Anorganischen Chemie, 91.-100 Aufl , W de Gruyter Verlag, Berlin, New York, 1985, S 408, Fußnote 50) oder um beliebige Mischungen letzterer mit überschüssigem Fluorid einerseits bzw. HF andererseits.

Es ist zur Herstellung der erfindungsgemäßen Trimerisate bzw Trimerisatmischungen unwesentlich, ob. der Katalysator im zu trimerisierenden Mono- bzw Polyisocyanat löslich ist (Homogenkatalyse), oder nicht (Heterogenkatalyse). Auch können weitere Stoffe bzw. Stoffgemische bei der Katalyse zugesetzt werden, z B. Amine, Alkohole, Phenole etc., Lösungsmittel für den Katalysator und/oder das Isocyanat, Antioxydanzien, sowie Matrizes zur adsorptiven oder kovalenten Bindung des Katalysators. Auch kann der zur Bildung der Hydrogen(poly)fluoride nötige Fluorwasserstoff separat, gegebenenfalls in gelöster Form, dem zu trimerisierenden Isocyanat(gemisch) vor oder während der Trimerisierung zugefügt werden. Weiterhin können beliebige Substanzen, die Fluorwasserstoff unter den Katalysebedingungen liefern, zur Herstellung der erfindungsgemäßen Produkt(gemische) (mit)verwendet werden, so eignen sich beispielsweise beliebige Carbamoylfluoride als 'HF-Quelle' zur Herstellung der erfindungsgemäßen Trimerisate bzw. Trimerisatmischungen.

Die Katalyse kann in einem Temperaturbereich von -80°C bis +550°C in kondensierter Phase oder in der Gasphase, z.B. durch quantitative Umsetzung der beteiligten Isocyanatgruppen des/der Ausgangs(poly)isocyanat(es)/mischung erfolgen oder bei beliebigen Umsetzungsgraden unterbrochen werden. In letzterem Falle bieten sich zur Abstoppung der Reaktion, d.h. zur Inaktivierung des Katalysatorsystems, prinzipiell alle vorbeschriebenen Methoden des Standes der Technik an, wie z.B. die Zugabe (unter)stöchiometrischer Mengen an Säuren oder Säurederivaten (z.B. Benzoylchlorid, saure Ester der phosphorigen Säure und der Phosphorsaure, diese Säuren selbst etc., nicht jedoch HF), adsorptive Bindung des Katalysators und anschließende Abtrennung durch Filtration, thermische Desaktivierung usw. Zur Herstellung der erfindungsgemäßen Trimeren(mischungen) genügen Katalysatorkonzentrationen, bezogen auf eingesetzte(s) (Poly)-isocyanat(mischung), zwischen wenigen ppm und 5 %

Nach einer besonderen, gegebenenfalls kontinuierlich arbeitenden, Ausführungsform des Verfahrens kann die Herstellung der erfindungsgemäßen Trimeren(mischungen) in einem Rohrreaktor vorgenommen werden. Hierbei profitiert man zusätzlich von der geringeren Exothermie der erfindungsgemäßen Trimerisierung im Vergleich zur Isocyanuratbildung. Die erfindungsgemäße Trimerisierung kann gegebenenfalls unter simultaner Urethanisierung und/oder Allophanatisierung durchgeführt werden.

Die erfindungsgemäßen Trimeren(mischungen) können auch bei der Herstellung (Vernetzungsreaktion), gegebenenfalls geschäumter, Polyurethankunststoffe (mit)entstehen.

Zur Herstellung der erfindungsgemäßen Trimerisate bzw. Trimerisatmischungen können alle bekannten aliphatischen, cycloaliphatischen araliphatischen sowie aromatischen Mono- sowie Polyisocyanate eines NCO-Gehaltes von weniger als 70 % in reiner Form oder als beliebige Mischungen untereinander, verwendet werden. Beispielhaft seien genannt: Ethylisocyanat, alle Regio- und Stereoisomeren der nachstehend genannten Mono- sowie Polyisocyanate: Propylisocyanate, Butylisocyanate, Hexylisocyanate, Octylisocyanate, Alkoxyalkylisocyanate wie z.B. Methoxypropylisocyanat, Cyclohexylisocyanat, (Methyl)-cyclohexandiisocyanate, Ethylcyclohexandiisocyanate, Propylcyclohexandiisocyanate, Methyl-diethyl-cyclohexandiisocyanate, Phenylisocyanat, Phenylendiisocyanate, Tolylisocyanate, Toluylendiisocyanate, Bis(isocyanatophenyl)methan und Polyphenyl-polymethylen-polyisocyanate, wie sie beispielsweise durch Anilin-Formaldehyd-Kondensation und anschließende Phosgenierung hergestellt werden (MDI), Propandiisocyanate, Butandiisocyanate, Pentandiisocyanate, Hexandiisocyanate (z.B. HDI), Heptandiisocyanate, Octandiisocyanate, Nonandi- und triisocyanate, Dekandi- und triisocyanate, Undekandi- und triisocyanate, Dodecandi- und triisocyanate, Isophorondiisocyanat (IPDI), Bis(isocyanatocyclohexyl)methan (H₁₂MDI, Desmodur® W), Isocyanatomethyl-methylcyclohexane (z.B. 4(3)-Isocyanatomethylcyclohexylisocyanat: 'IMCI'). Hierbei ist es belanglos, nach welchen Verfahren die vorstehend genannten (Poly)isocyanate generiert werden, d.h. mit oder ohne Verwendung von Phosgen.

Es kann weiterhin von Vorteil sein, Gemische bestimmter (Poly)isocyanate in der erfindungsgemäßen Trimerisierungsreaktion einzusetzen, beispielsweise um dem Anforderungsprofil des jeweiligen Produktes bzw. Produktgemisches optimal zu entsprechen. So werden in vielen Anwendungen, beispielsweise bei der Automobil(erst)lackierung, Gemische von Isocyanat-Polyisocyanaten auf Basis, gegebenenfalls verzweigter, lineraliphatischer (z.B. HDI) einerseits und cycloaliphatischer Diisocyanate (z.B. IPDI, H₁₂MDI) andererseits eingesetzt. Diese Gemische werden in der Regel durch nachträgliches Abmischen reiner Isocyanurat-Polyisocyanate auf Basis von Diisocyanaten des einen mit denen des anderen Typs hergestellt. Es kann aber auch von Vorteil sein, sie durch echte Mischtrimerisation herzustellen (EP-A 0 047 452). Da aber insbesondere Isocyanat-Polyisocyanate des Standes der Technik auf Basis cycloaliphatischer Diisocyanate bereits bei Harzausbeuten unter 20 % fest sind und zuweilen eine derart hohe Schmelzviskosität aufweisen, daß eine Monomerenabtrennung durch (Dünnschicht)destillation erhebliche Schwierigkeiten bereitet, bedarf es zu ihrer Verarbeitung der Verwendung von Lösungsmitteln und mitunter auch von Fließhilfsmitteln bei der Dünnschichtdestillation. Will man keine allzu großen Einbußen in Umsetzungsgrad (Harzausbeute) und F bei der Herstellung von Isocyanurat-Polyisocyanaten hinnehmen, sind Lösungskonzentrationen um 70 % an Isocyanurat-Polyisocyanat auf Basis cycloaliphatischer Diisocyanate bei η²³-Werten von 1000-10 000 mPas marktüblicher Stand der Technik.

Trimerisiert man hingegen Gemische linearer aliphatischer (z.B. HDI) und cycloaliphatischer Diisocyanate, z.B. IPDI, unter (teilweiser) Iminooxadiazindionbildung, erhält man auch bei Zimmertemperatur fließfähige Produkte η²³ ≤ 100 000 mPas) die zudem in Lösung eine drastisch schnellere Viskositätsabnahme bei steigendem Lösungsmittelanteil aufweisen, als bekannte Produkte entsprechender Zusammensetzung (F, Diisocyanatbasis, mittleres Molekulargewicht loc. cit. Beispiel 4).

Auch sind die erfindungsgemäßen Trimerisate auf der Basis, gegebenenfalls verzweigter, reiner aliphatischer Diisocyanate, z.B. HDI, z.T. erheblich niedrigerviskos als entsprechende bekannte Produkte (vgl. EP-A 0 047 452 und loc. cit. Beispiel 5).

Die erfindungsgemäßen Trimeren(mischungen) können, gegebenenfalls im Gemisch mit anderen Isocyanatfolgeprodukten wie Urethan-, ('Prepolymer-'), Allophanat-, Harnstoff-, Biuret-, Uretdion- ('Dimerisat-') und/oder Oxadiazintrion-Strukturen enthaltenden Produkten anfallen und lassen sich durch die üblichen Verfahren des Standes der Technik isolierten, wie z.B. Dünnschichtdestillation, Extraktion, Kristallisation oder Molekulardestillation. Sie fallen dabei als farblose oder schwach gefärbte Flüssigkeiten oder Feststoffe an. Letztere weisen, abhängig von den eingesetzten Isocyanat(gemisch)en, einen Schmelzbereich von ca. 30-180°C auf.

Ein weiterer Vorteil der erfindungsgemäßen Imonooxadiazindione liegt in der Reaktivität des heterocyclischen Ringsystems begründet. Wesentliche Beiträge hierzu haben schon Slotta und Tschesche am Beispiel des Trimethylderivates **B**, R¹-R³ = Me, in der weiter oben bereits zitierten Arbeit und in Chem. Ber., 1927 60, 295 geliefert.

So reagiert die Verbindung mit Wasser unter Ringöffnung und Decarboxylierung zu 1,3,5-Trimethylbiuret, einer biologisch leicht abbaubaren Verbindung. Die Alkoholyse bzw. Aminolyse, unseres Wissens bisher nicht beschrieben, liefern Harnstoff-3-(carbonsäure-amid)-1-(carbonsäureester) bzw. Triurete.

Diese Verbindungsklassen sind auf alternativen Wegen nur schwer zugänglich (vgl. A. Botta in 'Houben/Weyl, Methoden der Organischen Chemie' Ergänzungsund Folgebände zur 4. Aufl., Bd. E4, Kohlensäure-Derivate, G. Thieme Verlag, Stuttgart, New York, 1983 Hrsg. H. Hagemann, S. 1325-1334) und können sowohl auf dem Wirkstoff- als auch auf dem Polyurethansektor interessant sein.

So eröffnet sich insbesondere in letzterem Applikationsfeld hiermit die Möglichkeit, wertvolle NCO-Gruppen, die in der Trimerisierungsreaktion zunächst für die Vernetzung zum hochmolekularen Kunststoff bzw. zur Beschichtung 'verlorengingen', wieder nutzbar zu machen. Führt man diese Reaktionen beispielsweise mit Iminooxadiazindionen **B,** die in den Substituenten R¹-R³ noch NCO-Gruppen enthalten, und (poly)hydroxyfunktionellen Produkten wie Polyethern oder Polyestern durch, kann die Ringöffnungs- und gegegebenenfalls auch die anschließende Dissoziationsreaktion der Tricarbonylverbindungen gezielt genutzt werden, um zu Isocyananurat-freien Kunststoffen, Beschichtungsmitteln oder Zuschlagstoffen zu gelangen, die hohen Anforderungen bezüglich der biologischen Abbaubarkeit gerecht werden. Derartige Produkte sind beispielsweise bei der Naßfestausrüstung von Papier von besonderem Interesse. Außerdem erhöht sich die formale NCO-Funktionalität F idealer Diisocyanat-Trimerer der allgemeinen Formel **A** bzw. **B**, R¹-R³ = R'-NCO wobei R' ein NCO-Gruppen-freier organischer Rest ist, von 3 bei **A** auf bis zu 5 im Falle von **B**.

Ein weiterer Vorteil der erfindungsgemäßen Iminooxadiazindione liegt in der Isomerisierung zu **A** begründet. So gelingt es in einfacher Weise bereits bei Zimmertemperatur und z.T. auch weit darunter, simultan zur Umsetzung der NCO-Gruppen mit Zerevitinoff-aktiven Wasserstoff enthaltend Verbindungen, gegebenenfalls in Gegenwart von Katalysatoren, die Umlagerung der Iminooxadiazindionstruktur in die isomere Isocyanuratstruktur vorzunehmen und so zu Kunststoffen und Beschichtungen zu gelangen, die dem hohem Eigenschaftsniveau von Isocyanurat-Polyisocyanaten des Standes der Technik in nichts nachstehen, vor und bei der Applikation jedoch beispielsweise die weiter oben angesprochenen Viskositätsvorteile bieten.

Die erfindungsgemäßen Verbindungen bzw. Gemische stellen mithin vielseitig verwendbare (Ausgangsmaterialien zur Herstellung von) Wirkstoffe(n), gegebenenfalls geschäumte(n), Kunststoffe(n) sowie Lacken, Beschichtungsmitteln, Klebstoffen und Zuschlagstoffen dar. Insbesondere zur Herstellung von Ein- und Zweikomponenten-Polyurethanlacken eignen sie sich, gegebenenfalls in NCOblockierter Form, aufgrund ihrer im Vergleich zu (überwiegend) Isocyanurat-Polyisocyanat basierenden Produkten verringerten Lösungs- sowie Schmelzviskosität bei ansonsten gleich hohem bzw. verbessertem Eigenschaftsprofil.

Im letzterem Applikationsfeld können sie, rein oder in Verbindung mit anderen Isocyanatderivaten des Stand der Technik, wie Uretdion-, Biuret-, Allophanat-, Isocyanurat-, Urethan- sowie Carbodiimid-Polyisocyanaten, deren freie NCO-Gruppen gegebenenfalls mit Blockierungsmitteln desaktiviert wurden, eingesetzt werden.

Ein weiterer Vorteil der erfindungsgemäßen Trimeren(mischungen) ist darin zu sehen, daß sie selbst bei längerer thermischer Belastung keine Rückspalttendenz in die zugrundeliegenden monomeren (Poly)isocyanate aufweisen. So lassen sich selbst so hochsiedende Verbindungen wie das Tris(6-isocyanatohexyl)iminooxadiazindion **B,** R¹-R³ = (CH₂)₆NCO, ohne Zersetzung oder Umlagerung zum isomeren Isocyanurat **A,** R¹-R³ = (CH₂)₆, zu erleiden, sowohl destillativ als auch extraktiv aus den erfindungsgemäßen HDI-Trimerenmischungen abtrennen. Hierbei resultieren Produkte, die die literaturbekannte Viskosität des 1,3,5-Tris(6-isocyanatohexyl)isocyanurates **A,** R¹-R³ = (CH₂)₆NCO, von 700 mPas wesentlich unterschreiten (loc. cit. Beispiel 6). Das Tris(6-isocyanatohexyl)iminooxadiazindion **B,** R¹-R³ = (CH₂)₆NCO, ist mithin das am niedrigsten viskose, NCOtrifunktionelle Oligomer des Hexamethylendiisocyanates.

Die erfindungsgemäßen Polyisocyanat-Trimeren(mischungen) eignen sich ebenfalls für Anwendungen mit dualem Vernetzungsmechanismus. Hierfür setzt man beispielsweise die freien reaktiven Gruppen, in der Regel Isocyanatgruppen, in einem ersten Reaktionsschritt mit einer Polyol- bzw Polyaminkomponente um und führt in einem unabhängigen zweiten Schritt eine weitere Vernetzung unter Abbau der Iminooxadiazindionstruktur durch Hierbei zeigte sich, wie bereits weiter oben erwähnt, daß bis zu zwei Reaktionspartner, die Zerevitinov-aktiven Wasserstoff enthalten, je Equivalent Iminooxadiazindioneinheit gebunden werden konnen.

Die resultierenden Kunststoffe und Beschichtungen entsprechen in ihrer chemischen Natur weigehend denen, die auf Basis Biuret- bzw. Allophanatgruppen enthaltender (Lack)rohstoffe (cyclo)aliphatischer Diisocyanate einerseits bzw. Isocyanuratgrupen enthaltender (Lack)rohstoffe andererseits, erhalten werden und sind somit außerordentlich hochwertige Produkte mit dem für die genannten, bewährten Systeme des Standes der Technik typischen Eigenschaftsprofil, ohne jedoch die bereits angesprochenen Nachteile aufzuweisen.

Die erfindungsgemäßen Polyisocyanat-Trimeren(mischungen) eignen sich für den Einsatz als Bindemittel in Beschichtungsmaterialien. Vorzugsweise werden sie, gegebenenfalls in Abmischung mit anderen typischen Rohstoffen des Standes der Technik, gegebenenfalls in blockierter Form, als Vernetzerkomponente in, gegebenenfalls wäßrigen, 1- und 2-K-Beschichtungen eingesetzt. Bei der Verwendung als Vernetzerkomponente in 2K-Beschichtungen werden die erfindungsgemäßen Polyisocyanate in der Regel mit OH- sowie NH-Komponenten kombiniert, wie sie aus 2K-Polyurethansystemen an sich bekannt sind, so z.B. hydroxyfunktionellen Polyestern, Polyacrylaten, Polycarbonaten, Polyethern, Polyurethanen sowie polyfunktionellen Aminen.

Sie können aber auch einkomponentig zur Herstellung (anteilig) feuchtigkeitshärtender Kunststoffe und Beschichtungen verwendet werden

Neben den erfindungsgemäßen Verfahrensprodukten und den gegebenenfalls mitverwendeten weiteren Bindemittelkomponenten und Lacklösungsmitteln oder Lacklösungsmittelgemischen wie beispielsweise Toluol, Xylol, Cyclohexan, Chlorbenzol, Butylacetat, Ethylacetat, Ethylglykolacetat, Methoxypropylacetat, Aceton, Testbenzin, höher substituierte Aromaten (Solventnaphtha^{R}, Solvesso^{R}, Schellsol^{R}, Isopar^{R}, Nappar^{R}, Diasol^{R}) können in den Beschichtungen auch weitere Hilfs- und Zusatzmittel verwendet werden, wie z.B. Benetzungsmittel, Verlaufsmittel, Hautverhinderungsmittel, Antischaummittel, Mattierungsmittel, viskositätsregulierende Stoffe, Pigmente, Farbstoffe, UV-Absorber, Katalysatoren und Stabilisatoren gegen thermische und oxidative Einflüsse.

Die Polyisocyanate auf Basis der erfindungsgemäßen Trimeren(mischungen) können zur Beschichtung bzw. als Zuschlagstoff zur Ausrüstung einer Vielzahl von Materialien dienen, wie z.B Holz, Kunststoff, Leder, Papier, Beton, Mauerwerk, Keramik und Textil.

### Beispiele

Alle Prozentangaben sind, soweit nicht anders vermerkt, als Gewichtsprozent zu verstehen. Die dynamischen Viskositäten wurden bei 23°C mit dem Viskosimeter VT 550, Platte-Kegel Meßanordnung PK 100, der Fa. Haake bestimmt. Durch Messungen bei unterschiedlichen Schwergeschwindigkeiten wurde sichergestellt, daß das Fließverhalten der beschriebenen erfindungsgemäßen Polyisocyanatmischungen wie auch das der Vergleichsprodukte dem idealer Newtonscher Flüssigkeiten entspricht. Die Angabe der Schwergeschwindigkeit kann deshalb entfallen.

### Beispiel 1: Vergleichsbeispiel zur Phosphinkatalyse (nicht erfindungsgemäß)

Jeweils 200 g (1,19 mol) frisch destilliertes HDI werden bei 60°C zunächst im Vakuum (0,1 mbar) zur Entfernung gelöster Gase 1h gerührt, anschließend mit trockenem Stickstoff belüftet und bei
a) 60°C
b) 120°C bzw.
c) 180°C,
mit jeweils 3 g (14,8 mmol) Tri-n-butylphosphin (Firma Acros, ehem. Janssen) versetzt und bis zum Erreichen des in Tab. 1 angegebenen Brechnungsindex der Rohlösung unter einer Stickstoffatmosphäre umgesetzt Anschließend wird durch Zugabe von jeweils 4 g (26 mmol) p-Toluolsulfonsäuremethylester die Weiterreaktion sicher unterbunden ('abgestoppt'), wozu jeweils ca. eine Stunde bei 80°C nachgerührt wird, bis sich der Brechnungsindex der Mischung nicht mehr ändert (vgl. Tab. 1).

Die Rohprodukte werden anschließend durch Dünnschichtdestillation bei 120°C/0,1 mbar in einem Kurzwegverdampfer vom nicht umgesetzten Monomer befreit. Anschließend wird die Produktzusammensetzung NMR-spektroskopisch und der Restmonomergehalt gaschromatographisch bestimmt. Letzterer wird nach 3-wöchiger Lagerung bei Zimmertemperatur (20-25°C) und anschließender 2-wöchiger Lagerung bei 50°C im Trockenschrank nochmals bestimmt. Sämtliche Analysenergebnisse sind in Tabelle 1 zusammengestellt.

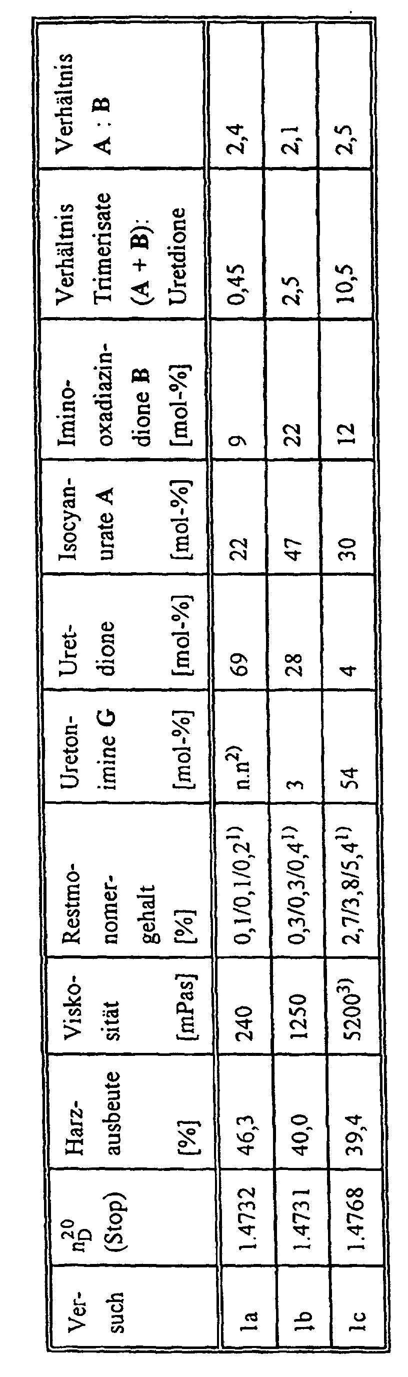

**Tab. 1**

| Resultate der Tributylphosphin-katalysierten HDI-Oligomerisierung bei verschiedenen Temperaturen | |
|---|---|
| 1) | Restmonomergehalt nach -Aufarbeitung/-3-wöchiger Lagerung bei Zimmertemperatur/-weiterer 2-wöchiger Lagerung bei 50°C |
| 2) | n.n. = nicht nachweisbar, |
| 3) | heterogenes, trübes Produkt |

### Beispiel 2: Vergleichsbeispiel zur Fluoridkatalyse (nicht erfindungsgemäß)

Jeweils 200 g (1,19 mol) frisch destilliertes HDI werden bei 60°C zunächst im Vakuum (0,1 mbar) zur Entfernung gelöster Gase 1h gerührt, anschließend mit trockenem Stickstoff belüftet und wie folgt weiter behandelt:
a) es werden bei 80°C ca. 900 ppm, bezogen auf Katalysator der Cg-Stöchiometrie und eingesetztes HDI, einer ca. 8 %igen Katalysatorlösung von Aliquat F⁻ in 2-Ethyl-1,3-hexandiol (hergestellt wie in der DE-A 3 902 078, Beispiel 1, beschrieben) zugegeben, wobei die Temperatur bis auf 105°C ansteigt und bis zum Erreichen eines NCO-Gehaltes von 41,2 % gerührt. Anschließend wird durch Zugabe von 0,9 g Phosphorsäuredi-n-butylester abgestoppt, eine weitere Stunde bei 60°C gerührt und anschließend durch Dünnschichtdestillation bei 120°C/0,1 mbar in einem Kurzwegverdampfer vom nicht umgesetzten Monomer befreit. Anschließend wird die Produktzusammensetzung und der Restmonomergehalt wie in Beispiel 1 bestimmt;
b) es wird verfahren wie bei a) angegeben, mit dem Unterschied, daß als Katalysator 110 ppm einer 5 %igen Katalysatorlösung von Tetramethylammoniumfluorid-Tetrahydrat (Fa. Aldrich) in n-Butanol verwendet werden, die Reaktion im Temperaturbereich von 60 - 70°C durchgeführt wird, bis zu einem NCO-Gehalt von 39,1 % trimerisiert und durch Zugabe von 0,132 g Phosphorsäuredi-n-butylester abgestoppt wird;
c) es wird verfahren wie bei a) angegeben, mit dem Unterschied, daß als Katalysator 190 ppm einer 8,3 %igen Lösung von Tetraethylammoniumfluorid-Hydrat (Fa. Aldrich) in n-Butanol verwendet werden, im Temperaturbereich von 70-150°C bis zu einem NCO-Gehalt von 39,9 % trimerisiert und durch Zugabe von 0,312 g Phosphorsäuredi-n-butylester abgestoppt wird;
d) es wird verfahren wie bei a) angegeben, mit dem Unterschied, daß als Katalysator 160 ppm einer 5 %igen Lösusng von Benzyltrimethylammoniumfluorid-Hydrat (Fa. Aldrich) in 2-Ethyl-1,3-hexandiol (Fa. Janssen) verwendet werden, bis zu einem NCO-Gehalt von 35,1 % trimerisiert und durch Zugabe von 0,03 g Phosphorsäuredi-n-butylester abgestoppt wird.

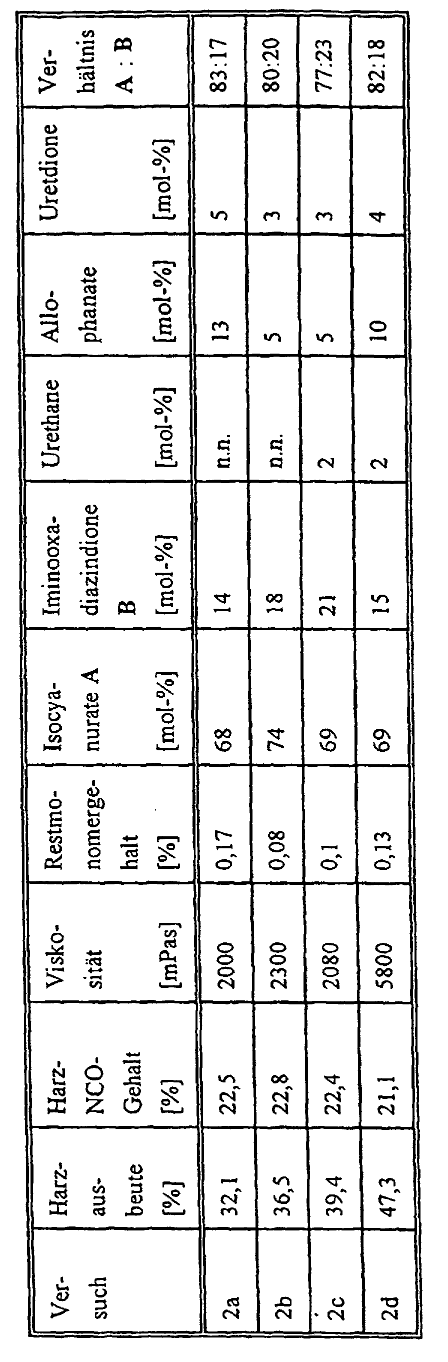

**Tab. 2**

| Die Resultate der Fluorid-katalysierten Trimerisierung von HDI |
|---|
| Die so hergestellten Produkte weisen entweder im Rohprodukt oder im monomerenfreien Harz häufig Trübungen auf, so daß vor oder nach der Dünnschichtdestillation filtriert werden muß. Nach längerer Lagerung der Harze, auch wenn sie vor bzw. nach der Dünnschichtdestillation filtriert worden waren, traten häufig erneut Trübungen auf. Wie Tabelle 2 zu entnehmen ist, liegt der molare Anteil von B im Trimerisatgemisch (Summe aus **A** und **B**) immer weit unter 30 %. |

### Beispiel 3 (Vergleichsbeispiel)

### Jeweils 1500 g eines

a) HDI-Isocyanurat-Polyisocyanates mit einem NCO-Gehalt von 23,5 % und einer Viskosität von 1380 mPas, hergestellt nach der Lehre der DE-A 3 806 276, bzw.
b) HDI-Oxadiazintrion-Polyisocyanates mit einem NCO-Gehalt von 22,5 % und einer Viskosität von 2560 mPas, hergestellt nach der Lehre der DE-A 1 670 666,
werden bei einem Druck von 0,05 mbar und einer Temperatur des Heizmediums von 220°C der Dünnschichtdestillation in einem Kurzwegverdampfer unterworfen.

Dabei gehen
a) 364 g bzw
b) 1092 g Destillat über, die anschließend jeweils bei 120°C mbar von monomerem HDI durch Dünnschichtdestillation befreit werden. Die so gereinigten Produkte weisen eine Viskosität von:

a) 700 ± 10 mPas bei 23°C bzw.
b) 1200 ± 20 mPas bei 23°C auf und bestehen nach Ausweis kombinierter analytischer Methoden (IR, NMR, GPC, MS) zu mindestens 98 % aus

a) dem idealen Isocyanurat-Trimeren des Hexamethylendiisocyanates (1,3,5-Tris(6-isocyanatohexyl)isocyanurat A, R¹-R³ = (CH₂)₆-NCO) bzw.
b) dem 3,5-Bis(6-isocyanatohexyl)-1-oxadiazintrion E, R¹ und R² = (CH₂)₆-NCO.

Die Messungen an A stehen völlig im Übereinklang mit literaturbekannten Daten (vgl. WO-A 93/07 183, die in den dort angegebenen Beispielen zitierten Viskositäten wurden bei 25°C an weniger reinen "Ideal-Isocyanaurat"-Fraktionen gemessen). Für E stehen keine Vergleichsangaben aus der Literatur zur Verfügung.

### Beispiel 4 (erfindungsgemäß)

Eine Mischung aus 84 g (0,5 mol) HDI und 111 g (0,5 mol) Isophorondiisocyanat (IPDI) wird in einem 250 ml Vierhalskolben mit Innenthermometer, Rührer, Rückflußkühler, Gaseinleitungsrohr sowie Dosiereinrichtung für die Katalysatorlösung zunächst bei Zimmertemperatur und einem Druck von ca. 0,1 mbar im Verlauf einer Stunde von im Diisocyanatgemisch gelösten Gasen befreit und anschließend unter Durchleiten eines schwachen Stickstoffstromes auf 60°C Innentemperatur erhitzt. Anschließend werden bei dieser Temperatur im Verlaufe von ca. 20 Minuten portionsweise ingesamt 1.614 g (920 ppm) einer Losung von 0,5 g Tetraethylammoniumfluorid-Hydrat (Fa. Aldrich) und 0,2 g Fluorwasserstoff in 5,6 g 2-Ethyl-1,3-hexandiol so zugesetzt, daß die Innentemperatur 70°C nicht überschreitet. Anschließend wird bei 60-70°C bis der NCO-Gehalt der Mischung bei 34,2 % liegt trimerisiert, durch Zugabe von 0,181 g Di-n-butylphosphat abgestoppt, eine weitere Stunde bei 60°C nachgerührt und anschließend von nicht umgesetzten monomeren Diisocyanaten durch Dünnschichtdestillation in einem Kurzwegverdampfer bei 0,1 mbar und einer Temperatur des Heizmediums von 170°C abgetrennt. Die Zusammensetzung des abdestillierten Diisocyanatgemisches beläuft sich auf 65 mol-% IPDI und 35 mol-% HDI.

Das so erhaltene, klare und nahezu farblose Harz (62,4 g entsprechend 32 % Ausbeute) weist in reiner Form eine Viskosität von 26 500 mPas, 18,8 % NCO-Gehalt und Restmonomergehalte von 0,13 % an HDI und 0,27 % an IPDI auf. Das molare Verhältnis von Isocyanuraten **A** zu Iminooxadiazindionen **B** belauft sich auf 50:50 %, wobei R¹-R³ für difunktionelle Alkylreste stehen, wie sie durch Entfernung beider NCO-Gruppen des HDI und/oder des IPDI entstehen die ihrerseits endständig durch NCO-Gruppen, Isocyanurat-, Iminooxadiazindion-, Uretdionringe und/oder Urethan- bzw. Allophanatgrupen abgesättigt sind.

### Beispiel 5 (erfindungsgemäß)

2000 g HDI werden zunächst so vorbehandelt, wie in Bsp. 4 beschrieben, anschließend werden bei einer Innentemperatur von zunächst 50°C insgesamt 17,23 g (520 ppm, bezogen auf Katalysator der C₈-Stöchiometrie), einer 6 %igen Lösung von Aliquat[Fx5 HF]⁻ in 2-Ethyl-1,3-hexandiol (hergestellt wie in der DE-A 3 902 078, Beispiel 1, beschrieben; mit dem Unterschied, daß anschließend die entsprechende Menge HF, als separat bereitete Lösung in 2-Ethyl-1,3-hexandiol, zugegbeben wird) portionsweise über einen Zeitraum von 90 min so zugetropft, daß die Innentemperatur 65°C nicht überschreitet. Bei einem NCO-Gehalt der Mischung von 40 % werden 0,22 g Dibutylphosphat zugegeben, eine weitere Stunde bei 50°C nachgerührt und anschließend aufgearbeitet wie unter Beispiel 4 beschrieben. Man erhält 720 g entsprechend 36 % Harzausbeute einer farblosen, klaren Trimerisatmischung die folgende Daten aufweist:
NCO-Gehalt: 22,8 %
Viskosität: 1490 mPas
Restmonomergehalt: 0,17 % HDI
molares Verhältnis **A**:**B**: 50:50

### Beispiel 6 (erfindungsgemäß)

570 g des gemäß Beispiel 5 erhaltenen Produktes werden unter den gleichen Bedingungen, wie im Beispiel 3 aufgeführt destilliert und gereinigt. Man erhält 125 g einer mehr als 98 % reinen Trimerisatmischung (Summe aus **A** und **B,** R¹-R³ = (CH₂)₆-NCO) mit im Vergleich zur Ausgangsoligomerenmischung unverändertem Verhältnis von **A** zu **B** Die Viskosität dieser Mischung beträgt 380 mPas

## Patentansprüche

1. Isocyanat-Trimere(ngemische) auf der Basis aliphatischer, cycloaliphatischer, aromatischer bzw. araliphatischer Mono- sowie Polyisocyanate eines NCO-Gehaltes unter 70 %, die in den organischen Resten gegebenenfalls weitere Substituenten wie Carbonyl- oder Carboxylgruppen sowie Heteroatome bzw. Heteroatomgruppierungen (Halogen, O, S, N, P, Si, Sn, B) enthalten können, sowie beliebiger Mischungen dieser Isocyanate, **dadurch gekennzeichnet, daß** die Trimeren(mischungen) anteilig aus Verbindungen vom Isocyanurat (Formel **A**) sowie vom Iminooxadiazindion-Strukturtyp (Formel **B**) bestehen, wobei die molare Zusammensetzung der Trimeren(mischungen) im Bereich von 70 % **A** und 30 % **B** bis 100 % **B** liegen kann, daß das molare Verhältnis von Trimerisat (Summe aus **A** und **B**) zu Uretdion größer als 4:1 ist und daß die Produkte einen Gehalt an Uretoniminen G unter 10 mol-% aufweisen.

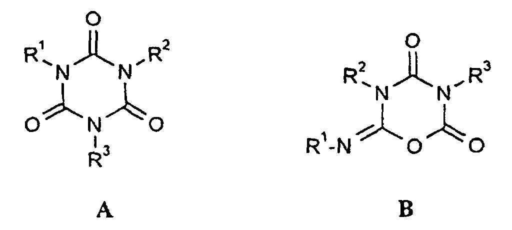

2. Mischungen der Trimeren(mischungen) nach Anspruch 1 mit weiteren Isocyanat-Folgeprodukten, beispielsweise vom Urethan-('Prepolymer-'), Allophanat-, Harnstoff-, Biuret-, Uretdion- ('Dimerisat') und/oder Oxadiazintrion-Strukturtyp, wobei diese ihrererseits in den organischen Resten weitere Substituenten, wie NCO-, Carbonyl- oder Carboxylgruppen sowie Heteroatome bzw. Heteroatomgruppierungen (Halogen, O, S, N, P, Si, Sn, B) enthalten können.

3. Verfahren zur Herstellung der Trimeren(mischungen) nach Anspruch 1 durch katalytisch induzierte Trimerisierung von Mono- und Polyisocyanaten eines NCO-Gehaltes unter 75 %, die in den organischen Resten gegebenenfalls weitere Substituenten wie Carbonyl- oder Carboxylgruppen sowie Heteroatome bzw Heteroatomgruppierungen (Halogen, O, S, N, P, Si, Sn, B) enthalten können.

4. Verfahren nach Anspruch 3, **dadurch gekennzeichnet, daß** Katalysatoren-(mischungen) auf der Basis von Hydrogen(poly)fluoriden der allgemeinen Zusammensetzung {M[nF⁻(HF)ₘ]}eingesetzt werden, wobei gilt ^{m}/ₙ > 0 und M ein n-fach geladenes Kation bzw. einen n-wertigen Rest darstellt.

5. Mono- bzw. Polyisocyanat-Trimeren(mischungen) nach Anspruch 1 die gegebenenfalls NCO-Gruppen, letztere gegebenenfalls auch in blockierter Form, enthalten können, als bzw. zur Herstellung von Wirkstoffe(n), gegebenenfalls geschäumte(n) Polyurethan-Kunststoffe(n), Lacken, Beschichtungsmitteln, Klebstoffen sowie Zuschlagstoffe(n).

## Claims

1. Isocyanate trimers (trimer mixtures) based on aliphatic, cycloaliphatic, aromatic and/or araliphatic mono and polyisocyanates having an NCO content of less than 70%, which may optionally contain in the organic radicals further substituents such as carbonyl or carboxyl groups and heteroatoms and/or heteroatom groups (halogen, O, S, N, P, Si, Sn, B), and on any mixtures of the said isocyanates, **characterised in that** the trimers (trimer mixtures) comprise proportionally compounds of the isocyanurate (formula **A**) and of the iminooxadiazine dione structural type (formula **B**), wherein the molar composition of the trimers (trimer mixtures) may be within the range 70% **A** and 30% **B** to 100% **B**, **in that** the molar ratio of trimer (sum of **A** and **B**) to uretdione is greater than 4 : 1, and **in that** the products have less than 10 mol.% uretone imines **G**.

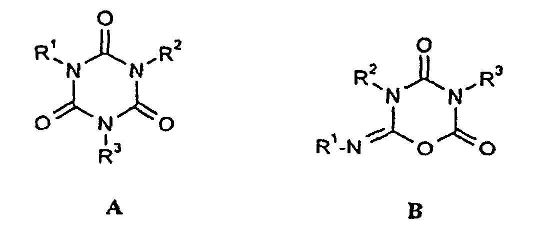

2. Mixtures of the trimers (trimer mixtures) according to Claim 1 having further isocyanate secondary products, for example of the urethane ("prepolymer"), allophanate, urea, biuret, uretdione ("dimer") and/or oxadiazine trione structural type, wherein these may for their part contain in the organic radicals further substituents such as NCO, carbonyl or carboxyl groups and heteroatoms and/or heteroatom groups (halogen, O, S, N, P, Si, Sn, B).

3. Process for the preparation of the trimers (trimer mixtures) according to Claim 1 by catalytically induced trimerisation of mono and polyisocyanates having an NCO content of less than 75%, which may optionally contain in the organic radicals further substituents such as carbonyl or carboxyl groups and heteroatoms and/or heteroatom groups (halogen, O, S, N, P, Si, Sn, B).

4. Process according to Claim 3, **characterised in that** catalysts (catalyst mixtures) based on hydrogen (poly)fluorides of the general composition {M[nF⁻ (HF)ₘ]} are utilised, wherein it is the case that ^{m}/ₙ > 0 and M is an n-charged cation and/or a n-valent radical.

5. Mono and/or polyisocyanate trimers (trimer mixtures) according to Claim 1 which may optionally contain NCO groups, the latter optionally also in blocked form, as, and/or for the preparation of, active ingredients and optionally foamed polyurethane plastics materials, for the preparation of lacquers, coating agents and adhesives and as, and/or for the preparation of, additives.

## Revendications

1. Trimères d'isocyanates (et mélanges) à base de mono- et poly-isocyanates aliphatiques, cycloaliphatiques, aromatiques ou araliphatiques à une teneur en NCO inférieure à 70 %, qui peuvent contenir le cas échéant dans les radicaux organiques d'autres substituants tels que des groupes carbonyle ou carboxyle ou des hétéroatomes ou groupements d'hétéroatomes (halogènes, O, S, N, P, Si, Sn, B) et de mélanges quelconques de ces isocyanates, **caractérisés en ce que** les trimères (et mélanges) consistent en partie en composés à structure d'isocyanurate (de formule A) et composés à structure d'imino-oxadiazinedione (de formule B), la composition molaire des trimères (et mélanges) pouvant aller de 70% de A et 30% de B jusqu'à 100% de B, le rapport molaire entre le trimère (somme de A et B) et l'uret-dione est supérieur à 4:1 et les produits ont une teneur en urétonimines G inférieure à 10 mol %.

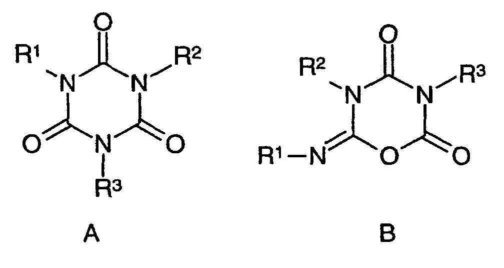

2. Mélanges des trimères (et mélanges) selon la revendication 1 avec d'autres produits de conversion des isocyanates, par exemple à structure d'uréthanne ("prépolymère"), d'allophanate, d'urée, de biuret, d'uret-dione ("dimère") et/ou d'oxadiazinetrione, qui peuvent contenir eux-mêmes dans les radicaux organiques d'autres substituants tels que les groupes NCO, carbonyle ou carboxyle ou des hétéroatomes ou groupements d'hétéroatomes (halogènes, O, S, N, P, Si, Sn, B).

3. Procédé pour la préparation des trimères (et mélanges) selon la revendication 1 par trimérisation catalytique de mono- et poly-isocyanate à une teneur en NCO inférieure à 75 % et qui peuvent contenir le cas échéant dans les radicaux organiques d'autres substituants tels que des groupes carbonyle ou carboxyle ou des hétéroatomes ou groupements d'hétéroatomes (halogènes, O, S, N, P, Si, Sn, B).

4. Procédé selon la revendication 3, **caractérisé en ce que** l'on utilise des catalyseurs (ou mélanges) à base d'hydrogéno(poly)fluorures de composition générale {M[nF(HF)ₘ]}, dans laquelle m/n > 0 et M représente un cation de charge n ou un radical de valence n.

5. Trimères (et mélanges) de mono- ou poly-isocyanate selon la revendication 1, qui peuvent contenir le cas échéant des groupes NCO, ces derniers éventuellement à l'état bloqué, pour l'utilisation dans ou pour la préparation de substances actives, de résines synthétiques de polyuréthanne éventuellement gonflées en mousse, de vernis, de produits de revêtement, de colles et d'additifs.
